(19) Europäisches Patentamt
European Patent Office
Office européen des brevets

(11) Publication number: **0 026 415**
**B1**

(12) # EUROPEAN PATENT SPECIFICATION

(45) Date of publication of patent specification: **30.05.84**

(21) Application number: **80105653.2**

(22) Date of filing: **19.09.80**

(51) Int. Cl.$^3$: **C 07 C 69/06**, C 07 C 67/40, C 07 C 67/36, B 01 J 23/72, B 01 J 23/86

(54) **Process for producing methyl formate by the catalytic conversion of methanol.**

(30) Priority: **20.09.79 US 77513**

(43) Date of publication of application:
**08.04.81 Bulletin 81/14**

(45) Publication of the grant of the patent:
**30.05.84 Bulletin 84/22**

(84) Designated Contracting States:
**BE DE FR GB NL**

(56) References cited:
**DE-A-2 710 726**
**US-A-1 400 195**

**Patents Abstracts of Japan, Vol. 4, No. 77, 4 June 1980, Page 34C13**
**Patents Abstracts of Japan, Vol. 2, No. 105, 30 August 1978, page 1932C78**

(73) Proprietor: **AIR PRODUCTS AND CHEMICALS, INC.**
**P.O. Box 538**
**Allentown, Pennsylvania 18105 (US)**

(72) Inventor: **MartinezdePinillos, Joaquin Victor**
**170 Pine Grove Circle**
**Wescosville, PA 18106 (US)**
Inventor: **DeLaMater, George Bearse**
**66 Hillcrest Drive Hillcrest Hats.**
**Macungie, PA 18062 (US)**
Inventor: **Ladenheim, Harry**
**459 Levering Mill Road**
**Bala cynwyd Pennsylvania 19004 (US)**

(74) Representative: **Berg, Wilhelm, Dr. et al**
**Dr. Berg, Dipl.-Ing. Stapf, Dipl.-Ing. Schwabe, Dr. Dr. Sandmair Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 026 415 B1**

## Description

Technical field

This invention relates to a process for producing methyl formate by the catalytic conversion of methanol at temperatures of from 149 to 260°C using a copper chromite catalyst.

Background of the prior art

The earliest work relating to the preparation of methyl formate by the catalytic dehydrogenation of methanol appears to be that of Mannich and Geilmann, Ber. 49,585-6 (1916). Other work includes:

A process for producing methyl formate by the catalytic dehydrogenation of methanol. Dehydrogenation is carried out by passing the methanol through a reactor heated by means of steam with the reactor being filled with a finely-divided catalyzer metal such as copper, nickel chromium or iron. Reactor temperatures are maintained at about 177—232°C with the reactor pressure presumably being atmospheric. Product is collected in an absorbing liquid; typically the methyl formate is recovered as a 35% solution in water.

Vapor phase conversion of alcohols to esters by catalytic dehydrogenation, the alcohols being limited to $C_2$ and above. The dehydrogenation is carried out under pressure, as opposed to atmospheric, with the result that a significant increase in yield and selectivity is achieved. Catalytic metals used for the dehydrogenation include copper, nickel, cobalt and iron or admixtures thereof. The dehydrogenation temperatures for $C_2$ and above alcohols are substantially higher than for methanol conversion.

A process for producing higher alkyl esters by the catalytic dehydrogenation of the corresponding alcohol. The catalysts used for the dehydrogenation are mutiple chromite catalysts, the catalysts containing a plurality of chromites of hydrogenating metals. Examples of such catalysts include cadmium-promoted zinc chromite catalysts, copper chromite catalysts and multicomponent catalysts such as one including zinc, copper and cadmium. The results show that the three component chromite catalyst is preferable to the two component system in producing the higher alkyl esters.

A process for forming methyl formate by the catalytic conversion of methanol in the presence of carbon monoxide, the reaction being carried out at superatmospheric pressures, i.e. from 200—700 bar and a reaction temperature of from 50—130°C. The catalyst used was an alkali metal alcoholate, e.g. sodium methylate or potassium ethylate.

Newer work in producing methyl formate involves the catalytic reaction of liquid methanol with gaseous carbon monoxide at a mole ratio of 0.05—20:1 in the presence of a strongly basic anion exchange resin. Temperatures used in the process are from about 0—200°C with carbon monoxide pressures of 6.8—344 bar.

JP—A—53-68716 discloses a process for producing methyl formate by the low pressure catalytic conversion of methanol at a temperature from 149 to 260°C and usually at atmospheric pressure using a copper chromite catalyst, whereby methyl formate is obtained with a conversion of about 22% and a selectivity of about 85% to methyl formate.

Brief summary of the invention

It has now been found that when carrying out the dehydrogenation of methanol in the presence of carbon monoxide a dramatical improvement of the conversion and the selectivity to methyl formate can be obtained. This invention therefore relates to a process for producing methyl formate by the low pressure catalytic conversion of methanol at temperatures of from 149—260°C using a copper chromite catalyst, which is characterized by employing a copper chromite catalyst having a proportion of copper oxide from 50—85%, a chromium oxide concentration of 45—10% and from 0—5% of a dehydrogenation metal oxide, the metal having an atomic number of 25 to 30; and carrying out said catalytic dehydrogenation at a pressure to 6.8 bar in the presence of carbon monoxide, the carbon monoxide being present in a proportion of 0.25—2 moles per mole of methanol.

The improvement resides in the utilization of relatively low pressures, e.g. from subatmospheric to 6.8 bar and the use of a copper chromite catalyst, the catalyst having a copper oxide concentration of from 50 to 85%, a chromium oxide concentration of from 45—10% and from 0—5% of another metal oxide capable of effecting conversion. Temperatures and pressures used during the catalytic conversion typically range from 149—260°C and pressures are from atmospheric to 3.4 bar, and broadly, subatmospheric to 6.8 bar.

Brief description of the drawing

The drawing is a process flow diagram for a methyl formate plant operating according to Examples 1, 2 and 4 not directly illustrating the invention and according to Example 3 of the invention. The recovery train is not shown.

Detailed description of the invention

The present invention relates to a method for preparing methyl formate by the low pressure catalytic conversion of methanol in the presence of carbon monoxide. Methyl formate is a clear, colorless volatile liquid having a boiling point of about 31.8°C and is used as an ingredient in

fumigants, or as a solvent in producing various organic chemicals, e.g. N,N-dimethylformamide or in producing acetic acid by isomerization.

The dehydrogenation of methanol to methyl formate proceeds by reaction 1 whereas the carbon monoxide addition to methanol proceeds by reaction 2.

Reaction 1 $2CH_3OH \rightarrow HCOOCH_3 + 2H_2$

Reaction 2 $CH_3OH + CO \rightarrow HCOOCH_3$

As the prior art indicates, methods have long existed for the preparation of methyl formate from methanol, but these methods generally result in poor yields or poor selectivity or both.

The catalytic reaction of this invention is carried out generally under conventional temperatures and pressures, e.g. 149—260°C, and preferably 177—232°C at pressures from atmospheric to 6.8 bar, but preferably atmospheric to about 3.4 bar. Higher yields in reaction 2 are produced by operating at lower pressures, implying a complex mechanism.

The catalyst used in carrying out the catalytic conversion is generally described as a copper chromite catalyst with the copper oxide concentration being from 50—85%, the chromium oxide being present in a proportion of from 45—10% and, optionally, the balance consisting of a dehydrogenation metal oxide and the like having an atomic number of from 25—30. These optional components, when used in small amounts, can provide beneficial effects to the overall catalyst, although good results are achieved with the two component copper-chromium catalyst system.

The catalyst generally is formed from a mixture of soluble salts, e.g. copper acetate, copper nitrate and the like with various chromium-containing salts, e.g. ammonium chromate, chromium acetate, and the like. These salts are mixed in the required amounts to provide a desired copper and chromium concentration and then dried and calcined in the presence of air at temperatures of from 300—600°C from 1 to 4 hours. A copper-chromite catalyst which is particularly effective in practicing the invention is sold by the Harshaw Co. and designated Cu 0203. It has a concentration of 80% copper oxide with the balance being chromium oxide.

The reactants are passed through the reactor at a rate to provide a liquid hourly space velocity (LHSV) of from 1 to 15. Conversion to methyl formate is best at a rate of 5 to 10. In a tubular reactor with a ratio of length/diameter (L/D) equal to 100/1, the LHSV translates to a linear velocity of 12 to 27 m/min or Reynolds number from 650—3500.

According to the invention, the yield and selectivity of the process to methyl formate are enhanced by the addition of carbon monoxide to the reaction medium. In view of this the methanol stream, as it is passed to the reactor, is diluted with carbon monoxide in a proportion of from

0.25—2 moles carbon monoxide per mole of methanol.

Preferably, the process utilizes a proportion of from

0.5 to 1.5 moles carbon monoxide per mole of methanol.

The following examples are provided to in more detail illustrate the invention and are not intended to restrict the scope thereof. While Examples 1, 2 and 4 are not directly illustrating the claimed invention, Example 3 provides a preferred embodiment of the invention. All parts are parts by weight and all percentages are expressed as weight percentages.

Example 1

Referring to the drawing, methyl formate is produced by the dehydrogenation of methanol via the plant process described.

Methanol, at a temperature 26.7°C and a pressure of 1.7 bar, is contained in methanol storage tank A. It is removed via line 1 at a rate of 1069 kg per hour and is charged to methanol feed tank B. There it is combined with methanol from line 2 obtained from the recovery part of the plant and then removed via line 30 to pump C, and then line 31 where it is split into streams 33 and 35, respectively. Approximately 9685 kg pounds methanol per hour are carried in line 31, and approximately 7212 kg are removed via line 33 to preheater D. In preheater D, the feed methanol is warmed and then conveyed to vaporizer E where it is heated to a temperature 123°C with steam. There, the pressure is increased to 6.7 bar. Methanol is then removed from vaporizer E via line 3 and passed to feed tower F. The feed is warmed against reactor product in feed tower F and then the warmed feed is introduced to tubular reactor G.

The tubular reactor contains approximately 2600 tubes each having an internal diameter of 2.54 cm and a length of approximately 6 m. These tubes are packed with Harshaw Cu-0203 copper chromite catalyst to provide a bed depth of about 15 m. The void fraction is approximately 0.35.

The methanol in line 4 from feed tower F is at a temperature of 199°C and a pressure of 6.39 bar and is introduced into reactor G at a rate to provide a linear gas velocity of 0.36 m per second at a design pressure drop of 0.1 bar. The LHSV is calculated to be 10. The reaction product is removed from reactor G through line 5 where it is cooled against incoming feed from line 3. The product is removed via line 32 and cooled further in gas cooler H and then sent to vent separator I. A gas stream 6 and a liquid stream 7 are removed and sent to the product recovery train.

The material balance with product composition is presented in Table 1. VAP and LIQ represents vapor and liquid respectively. CW represents cold water (21 °C) in the drawing.

TABLE 1

| Line number | 1 | 2 | 3 |
|---|---|---|---|
| Pressure bar | 1.7 | 1.7 | 6.7 |
| Temperature °C | 27 | 49 | 121 |
| Dew point °C | 79 | 79 | 121 |
| Flow rates moles | | | |
| $H_2$ | 0.0 | 0.0 | 0.0 |
| Carbon monoxide | 0.0 | 0.0 | 0.0 |
| Carbon dioxide | 0.0 | 0.0 | 0.0 |
| Methyl formate | 0.0 | 1.82 | 1.82 |
| Methanol | 73.44 | 592.97 | 495.24 |
| Water | 0.0 | 0.0 | 0.0 |
| Composition mol % | | | |
| $H_2$ (approx-chem) | 0.0 | 0.0 | 0.0 |
| Carbon monoxide | 0.0 | 0.0 | 0.0 |
| Carbon dioxide | 0.0 | 0.0 | 0.0 |
| Methyl formate | 0.0 | 0.3 | 0.3 |
| Methanol | 100.0 | 99.6 | 99.6 |
| Water | 0.0 | 0.0 | 0.0 |
| Flow rates kg/hr | | | |
| $H_2$ (approx-chem) | 0.0 | 0.0 | 0.0 |
| Carbon monoxide | 0.0 | 0.0 | 0.0 |
| Carbon dioxide | 0.0 | 0.0 | 0.0 |
| Methyl formate | 0.0 | 49 | 49 |
| Methanol | 1069 | 8635 | 7212 |
| Water | 0.0 | 0.0 | 0.0 |
| Composition weight % | | | |
| $H_2$ (approx-chem) | 0.0 | 0.0 | 0.0 |
| Carbon monoxide | 0.0 | 0.0 | 0.0 |
| Carbon dioxide | 0.0 | 0.0 | 0.0 |
| Methyl formate | 0.0 | 0.5 | 0.6 |
| Methanol | 100.0 | 99.4 | 99.3 |
| Water | 0.0 | 0.0 | 0.0 |
| Total flow mol/hr | 73.4 | 594.8 | 497.0 |
| kg/hr | 1069 | 8685 | 7262 |
| Actual $m^3$ | 0.0 | 0.0 | 16.3 |
| | LIQ | LIQ | VAP |

TABLE 1 (cont'd.)

| Line number | 4 | 5 | 6 | 7 |
|---|---|---|---|---|
| Pressure bar | 6.48 | 5.45 | 4.48 | 4.48 |
| Temperature °C | 88 | 210 | 49 | 49 |
| Dew point °C | 248.94 | 224.30 | 49 | — |
| Flow rates moles/hr | | | | |
| $H_2$ | 0.0 | 72.07 | 71.26 | 0.80 |
| Carbon monoxide | 0.0 | 0.70 | 0.69 | 0.00 |
| Carbon dioxide | 0.0 | 0.56 | 0.51 | 0.05 |
| Methyl formate | 1.82 | 36.31 | 6.49 | 29.82 |
| Methanol | 495.24 | 424.99 | 10.34 | 414.64 |
| Water | 0.0 | 0.0 | 0.0 | 0.0 |
| Composition mole % | | | | |
| $H_2$ (approx-chem) | 0.0 | 13.48 | 79.79 | 0.18 |
| Carbon monoxide | 0.0 | 0.13 | 0.77 | 0.00 |
| Carbon dioxide | 0.0 | 0.10 | 0.57 | 0.01 |
| Methyl formate | 0.3 | 6.7 | 7.2 | 6.6 |
| Methanol | 99.6 | 79.4 | 11.5 | 93.1 |
| Water | 0.0 | 0.0 | 0.0 | 0.0 |
| Flow rates kg/hr | | | | |
| $H_2$ (approx-chem) | 0.0 | 65.9 | 65.1 | 0.72 |
| Carbon monoxide | 0.0 | 19.6 | 19.4 | 0.2 |
| Carbon dioxide | 0.0 | 24.7 | 22.4 | 2.2 |
| Methyl formate | 109.5 | 2180.8 | 389.8 | 1790.9 |
| Methanol | 15867.6 | 13616.9 | 331.5 | 13285.3 |
| Water | 0.0 | 0.0 | 0.0 | 0.0 |
| Composition weight % | | | | |
| $H_2$ (approx-chem) | 0.0 | 0.9 | 15.8 | 0.0 |
| Carbon monoxide | 0.0 | 0.1 | 2.1 | 0.0 |
| Carbon dioxide | 0.0 | 0.1 | 2.4 | 0.0 |
| Methyl formate | 0.6 | 13.6 | 42.9 | 11.8 |
| Methanol | 99.3 | 85.1 | 36.5 | 88.0 |
| Water | 0.0 | 0.0 | 0.0 | 0.0 |
| Total flow mol/hr | 497.0 | 534.6 | 89.3 | 445.3 |
| kg/hr | 7247 | 7252 | 411 | 6840 |
| Actual $m^3$ | 21.7 VAP | 29.0 VAP | 4.0 VAP | 0.0 LIQ |

Table 1 shows the process provides a yield of about 13% methyl formate per pass and a product selectivity above 98%.

Example 2

Pilot plant studies were conducted to determine the effect that various process parameters have on the catalytic conversion of methanol to methyl formate (MeFO). The pilot plant utilized a tubular reactor having an internal diameter of 2.54 cm. The reactor was packed with Harshaw Cu-0203 copper chromite catalyst and the runs made at various temperatures and pressures. The liquid hourly space velocity was 5 in each of the runs. Table 2 provides the results.

TABLE 2
Conversion and selectivity to MeFo
vs. pressure and temperature

| T (°C) | P (bar) | % Conv.[1] | % selectivity[1] |
|---|---|---|---|
| 204 | 1 | 22.9 | 93.0 |
| 204 | 4.4 | 13.2 | 96.4 |
| 204 | 11.1 | 14.5 | 91.8 |
| 204 | 14.6 | 14.5 | 91.5 |
| 216 | 1 | 27.3 | 91.8 |
| 216 | 14.6 | 16.4 | 92.2 |
| 227 | 1 | 35.9 | 89.8 |
| 227 | 14.6 | 14.9 | 92.6 |

[1]Molar conversions and selectivities.

These results show that conversions of about 25—35% can be produced with the copper chromite catalyst at temperatures of 204—227°C. Higher conversion is noted at lower pressures, e.g. atmospheric.

When the above runs are repeated with copper filings and used in the prior art, some methyl formate is produced but the catalyst life is short. Other process problems are also noted.

Example 3

The procedure of Example 2 was repeated except that various feeds selected from methanol and methanol plus carbon monoxide were utilized. The pressure of the reaction was maintained at 1 bar and a temperature at 216°C with an LHSV of 5. Table 3 provides these results.

TABLE 3
Conversion and selectivity data

| Run<br>feed | 1<br>MeOH | 2<br>MeOH+CO | 3<br>MeFO | 4<br>MeFO+CO | 5<br>MeFO+MeOH |
|---|---|---|---|---|---|
| % Conversion | 18.3 | 57.1 | 36.5 | 49.3 | 1.22 |
| % Selectivity | | | | | |
| MeFO | 66.4 | 93.8 | — | — | — |
| MeOH | — | — | 91.6 | 99.6 | — |
| CO | 33.5 | — | 8.09 | — | 75.9 |
| $CO_2$ | 0.16 | 6.15 | 0.31 | 0.43 | 24.1 |
| *Feeds $R_1/R_2$ | — | 4.19 | — | 1.87 | 1.0 |
| *Out $R_1/R_2$ | — | 37.4 | — | 1.37 | 0.57 |

*$R_1$ and $R_2$ are the moles of each reactant as written, in the feed and in the product, respectively, e.g. Run 2—4.19 moles MeOH and 1 mole CO

P=1 bar
T=221°C

It can be seen from Table 3 that the addition of carbon monoxide to the feed methanol substantially increases the percent methanol converted to methyl formate per pass through the reactor as compared to the procedure of Example 2. In addition, the selectivity to methyl formate was as high as any reported, e.g. 93%.

Example 4

Several laboratory runs effecting dehydrogenation of methanol to methyl formate were carried out using a variety of copper chromite catalysts. The laboratory scale used 30 $cm^3$ of catalyst which was diluted with an equal quantity of tab alumina. The methanol feed contained 0.1% water, and the liquid hourly space velocity and the temperature from the inlet to exit was as indicated.

Table 4 provides these results.

Run 1 used 30 $cm^3$ of Harshaw CU-0203 copper chromite catalyst.

Run 2 used 30 $cm^3$ of 80% CuO and 20% $Cr_2O_3$.

Run 3 used 30 $cm^3$ of CuO-free copper chromite.

Run 4 used 30 $cm^3$ of copper chromite in a 1:1 mole ratio of metal.

Run 5 used 30 $cm^3$ of copper:chromium 1/1 M/M catalyst.

Run 6 used 30 $cm^3$ of copper hydrate and ZnO containing 80% CuO.

TABLE 4

| Run[a] | Day | LHSV | Temp.—°C[b] | Mole % MeOH conv. |
|---|---|---|---|---|
| 1 | 7th | 5.00 | 182,176 | 29.0 |
| | 14th | 5.02 | 180,175 | 27.1 |
| | 22nd | 5.05 | 181,175 | 27.3 |
| | 28th | 5.00 | 184,173 | 25.2 |
| | 43rd | 5.02 | 183,173 | 23.3 |
| | 49th | 4.92 | 182,170 | 22.0 |
| | 57th | 5.08 | 185,173 | 21.9 |
| | 63rd | 5.00 | 185,173 | 21.7 |
| | 70th | 4.90 | 184,173 | 21.8 |
| | 77th | 5.08 | 182,173 | 20.8 |
| 2 | 2nd | 5.02 | 173,177 | 24.8 |
| | 7th | 5.0 | 176,176 | 22.9 |
| | 14th | 4.98 | 174,174 | 18.6 |
| | 21st | 4.92 | 174,173 | 16.2 |
| | 28th | 5.03 | 177,176 | 15.3 |
| 3 | 8th | 4.98 | 173,174 | 24.5 |
| | 14th | 5.18 | 172,173 | 23.2 |
| | 22nd | 5.00 | 171,172 | 24.8 |
| | 28th | 4.95 | 174,174 | 24.8 |
| | 35th | 5.00 | 173,173 | 24.3 |
| | 41st | 5.03 | 172,171 | 23.4 |
| | 43rd | 5.02 | 228,231 | 49.5 |
| | 47th | 5.00 | 228,234 | 51.0 |
| | 51st | 5.05 | 222,230 | 42.7 |
| | 54th | 4.98 | 255,270 | 57.9 |
| | 57th | 5.00 | 258,272 | 54.9 |
| | 62nd | 5.00 | 173,173 | 19.4 |
| 4 | 2nd | 5.02 | 175,173 | 22.5 |
| | 8th | 5.02 | 174,170 | 19.2 |
| | 11th | 5.03 | 173,170 | 19.2 |
| | 15th | 5.00 | 172,167 | 17.6 |
| | 18th | 5.02 | 175,170 | 18.6 |
| | 25th | 4.97 | 172,166 | 16.3 |
| | 30th | 5.00 | 172,166 | 15.4 |
| 5 | 7th | 5.00 | 178,174 | 20.5 |
| | 11th | 5.03 | 180,176 | 20.9 |
| | 15th | 5.05 | 180,177 | 20.6 |
| | 18th | 4.98 | 164,162 | 14.7 |
| | 21st | 4.47 | 162,160 | 14.5 |
| | | Ave. | | |
| 6 | 2nd | 5.00 | 173,176 | 23.6 |
| | 6th | 5.00 | 172,174 | 18.7 |
| | 9th | 4.92 | 173,175 | 17.3 |
| | 13th | 4.92 | 173,175 | 13.9 |
| | 14th | 4.97 | 189,193 | 21.4 |

[a)] 30 $cm^3$ of copper chromite diluted with tabular alumina, except for Run 6
[b)] Temperature at inlet, and exit of bed.

TABLE 4 (cont'd.)

| Run[a] | Day | Mole % selectivity MF | CO | $CO_2$ | Mole % MF yield |
|---|---|---|---|---|---|
| 1 | 7th | 76.2 | 22.8 | 1.0 | 22.1 |
| | 14th | 79.2 | 19.6 | 1.2 | 21.5 |
| | 22nd | 79.6 | 19.8 | 0.6 | 21.7 |
| | 28th | 83.9 | 13.9 | 2.2 | 21.1 |
| | 43rd | 88.2 | 10.7 | 1.1 | 20.5 |
| | 49th | 88.3 | 10.6 | 1.1 | 19.4 |
| | 57th | 87.6 | 11.1 | 1.3 | 19.2 |
| | 63rd | 89.5 | 9.2 | 1.3 | 19.4 |
| | 70th | 89.1 | 9.5 | 1.4 | 19.4 |
| | 77th | 91.0 | 7.8 | 1.2 | 18.9 |
| 2 | 2nd | 80.7 | 18.7 | 0.6 | 20.0 |
| | 7th | 85.5 | 13.9 | 0.6 | 19.6 |
| | 14th | 89.9 | 9.4 | .7 | 16.7 |
| | 21st | 92.4 | 6.6 | 1.0 | 15.0 |
| | 28th | 93.1 | 5.9 | 1.0 | 14.2 |
| 3 | 8th | 70.8 | 20.8 | 2.4 | 17.3 |
| | 14th | 72.9 | 25.1 | 2.0 | 16.9 |
| | 22nd | 68.8 | 28.9 | 2.3 | 17.1 |
| | 28th | 71.7 | 26.8 | 1.5 | 17.8 |
| | 35th | 73.8 | 24.6 | 1.6 | 17.9 |
| | 41st | 74.8 | 23.4 | 1.8 | 17.5 |
| | 43rd | 22.6 | 74.7 | 2.1 | 11.2 |
| | 47th | 25.8 | 71.7 | 1.9 | 12.6 |
| | 51st | 46.1 | 51.5 | 1.9 | 19.7 |
| | 54th | 21.8 | 73.0 | 3.8 | 12.6 |
| | 57th | 24.2 | 71.1 | 3.5 | 13.3 |
| | 62nd | 88.8 | — | — | 17.2 |
| 4 | 2nd | 80.8 | 17.4 | 1.8 | 18.2 |
| | 8th | 85.2 | 12.7 | 2.1 | 16.4 |
| | 11th | 87.0 | 10.8 | 2.2 | 16.7 |
| | 15th | 89.4 | 8.5 | 2.1 | 15.7 |
| | 18th | 88.2 | 10.0 | 1.8 | 16.4 |
| | 25th | 90.2 | 7.2 | 2.6 | 14.7 |
| | 30th | 90.3 | 7.1 | 2.6 | 13.9 |
| 5 | 7th | 71.1 | 26.0 | 2.9 | 14.6 |
| | 11th | 69.7 | 27.3 | 3.0 | 14.6 |
| | 15th | 70.4 | 28.1 | 1.5 | 14.5 |
| | 18th | 80.3 | 17.2 | 1.5 | 11.8 |
| | 21st | 80.1 | 17.9 | 2.0 | 11.6 |
| 6 | 2nd | 97.1 | 2.9 | 2.9 | 22.9 |
| | 6th | 98.0 | 2.0 | 2.0 | 18.3 |
| | 9th | 98.2 | 1.8 | 1.8 | 17.0 |
| | 13th | 98.5 | 1.5 | 1.5 | 13.7 |
| | 14th | 97.4 | 2.6 | 2.6 | 20.8 |

[a]30 $cm^3$ of copper chromite diluted with tabular alumina, except for Run 6
[b]Temperature at inlet, and exit of bed.

On examination of the data, all of the catalysts were very effective for producing methyl formate with good yield and good selectivity. The Harshaw Cu-0203 copper chromite catalyst was the most effective in terms of providing an enhanced yield and extended catalyst life.

With respect to the catalysts, the catalyst in Run 2 consisted of an 80% copper oxide, 20% chromium oxide mixture prepared in conventional manner by the mixing of a copper hydrate and a sodium dichromate salt with ammonium hydroxide. The salts were precipitated, pelletized and then calcined. In Run 3 the catalyst used was a copper chromium catalyst free of copper oxide. The catalyst

was prepared in essentially the same manner as the copper chromite catalyst except that copper nitrate was used. Additionally, an acetic acid leach was used in preparing the catalyst system with the end product being essentially a 1.58 mm chromite wafer. Runs 4 and 5 utilized a catalyst composition with a copper to chromium metal content in a 1:1 molar ratio. The copper chromite catalyst was prepared in similar manner as the catalyst for Run 3, i.e., from a mixture of copper nitrate and sodium dichromate. An acetic acid leach was not used. Lastly, Run 6 pertained to a copper zinc oxide mixture, containing 80% copper oxide and a balance of zinc oxide. The material was prepared in conventional manner and pelleted from a copper hydrate-zinc oxide mixture.

Statement of industrial application

Advantages of the process include:

an ability to produce methyl formate in high yields, e.g. up to 50% when carbon monoxide is used, and with high selectivity, e.g. greater than 80%;

the ability to produce methyl formate product in anhydrous form, thereby permitting easier product purification; and

a process for producing methyl formate that permits outstanding catalyst life.

**Claims**

1. A process for producing methyl formate by the low pressure catalytic conversion of methanol at temperatures of from 149—260°C, using a copper chromite catalyst, characterized by employing a copper chromite catalyst having a proportion of copper oxide from 50—85%, a chromium oxide concentration of 45—10% and from 0—5% of a dehydrogenation metal oxide, the metal having an atomic number of 25 to 30; and carrying out said catalytic dehydrogenation at a pressure from subatmospheric pressure to 6,8 bar in the presence of carbon monoxide, the carbon monoxide being present in a proportion of 0.25—2 moles per mole of methanol.

2. The process of Claim 1, characterized in that said carbon monoxide is included in a proportion of from 0.5—1.5 moles per mole of methanol.

3. The process of Claim 1, characterized in that said catalyst contains 0.75 to 0.85 weight parts copper oxide and the balance being chromium oxide.

4. The process of Claim 1, characterized in that said catalyst is supported on diatomaceous earth.

**Patentansprüche**

1. Verfahren zur Herstellung von Methylformiat durch katalytische Niederdruck-Umwandlung von Methanol bei Temperaturen von 149—260°C unter Einsatz eines Kupferchromitakatalysators, dadurch gekennzeichnet, daß ein Kupferchromitkatalysator mit einem Anteil von 50 bis 85% Kupferoxid, einer Chromoxidkonzentration von 45 bis 10% und von 0 bis 5% eines Dehydrierungsmetalloxids, wobei das Metall eine Ordnungszahl von 25 bis 30 besitzt, verwendet wird und die katalytische Dehydrierung bei einem Druck von unteratmosphärischem Druck bis 6,8 bar in Gegenwart von Kohlenmonoxid durchgeführt wird, wobei das Kohlenmonoxid in einem Anteil von 0,25 bis 2 Mol pro Mol Methanol vorhanden ist.

2. Verfahren nach Anspruch 1, dadurch gekennzeichnet, daß das Kohlenmonoxid in einem Anteil von 0,5 bis 1,5 Mol pro Mol Methanol vorhanden ist.

3. Verfahren nach Anspruch 1, dadurch gekennzeichnet, daß der Katalysator 0,75 bis 0,85 Gewichtsteile Kupferoxid enthält und der Rest Chromoxid ist.

4. Verfahren nach Anspruch 1, dadurch gekennzeichnet, daß der Katalysator auf Diatomeenerde getragen wird.

**Revendications**

1. Procédé de fabrication de formiate de méthyle par conversion catalytique de méthanol à basse pression, à des températures de 149 à 260°C, en utilisant un catalyseur consistent en chromite de cuivre, caractérisé en ce qu'on utilise un catalyseur consistant en chromite de cuivre ayant une proportion d'oxyde de cuivre de 50 à 85%, une concentration en oxyde de chrome de 45 à 10% et de 0 à 5% d'un oxyde de métal déshydrogénant, ce métal ayant un nombre atomique de 25 à 30, et effectue la déshydrogénation catalytique à une pression comprise entre une pression inférieure à la pression atmosphérique et 6,8 bars, en présence de monoxyde de carbone, le monoxyde de carbone étant présent en une proportion de 0,25 à 2 moles par mole de méthanol.

2. Procédé suivant la Revendication 1, caractérisé en ce que le monoxyde de carbone est présent en une proportion de 0,5 à 1,5 moles par mole de méthanol.

3. Procédé suivant la Revendication 1, caractérisé en ce que le catalyseur contient 0,75 à 0,85 parts en poids d'oxyde de cuivre, le complément étant de l'oxyde de chrome.

4. Procédé suivant la Revendication 1, caractérisé en ce que le catalyseur est fixé sur de la terre diatomée.

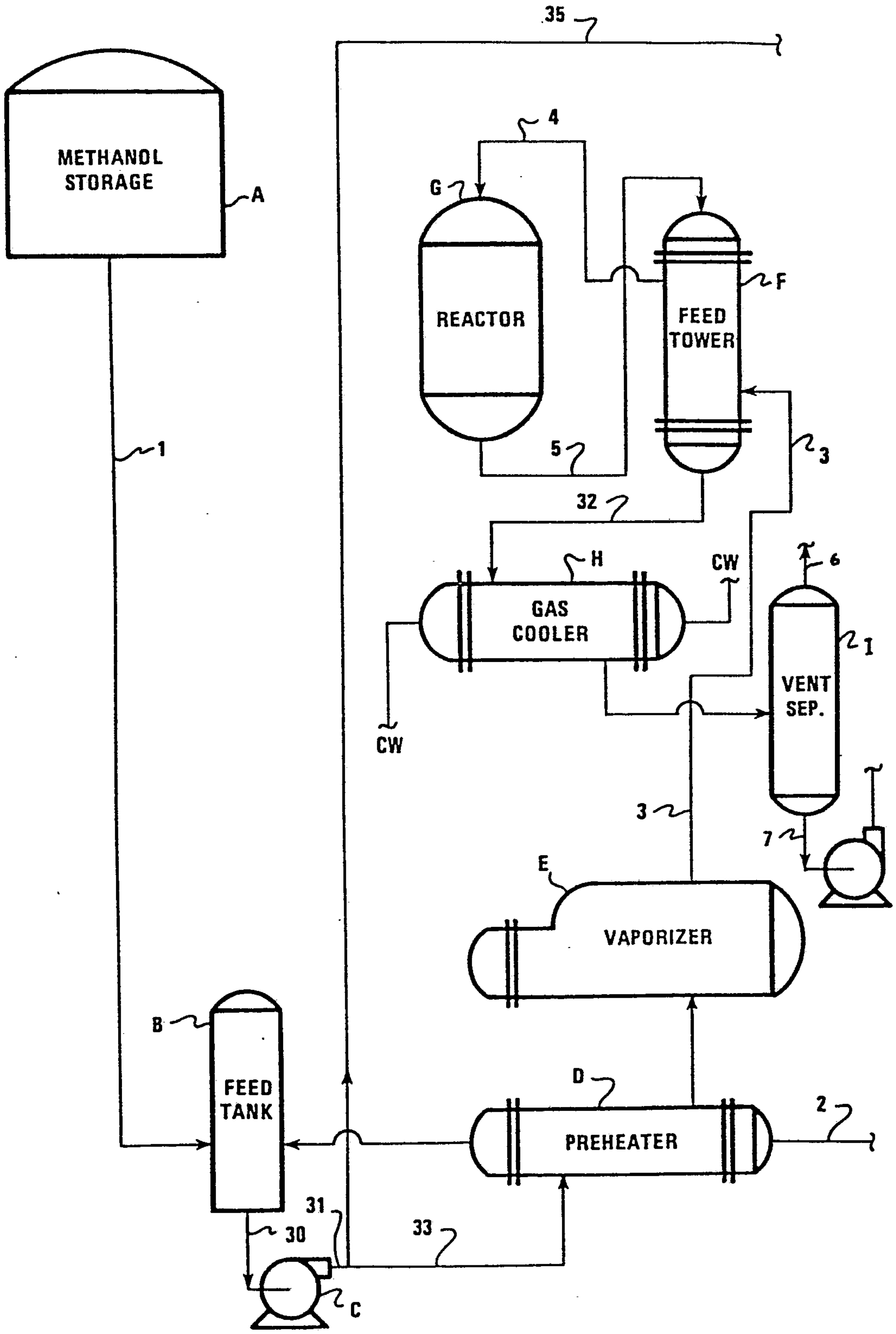
35
METHANOL STORAGE
A
4
G
REACTOR
F
FEED TOWER
1
5
3
32
H
CW
6
GAS COOLER
I
VENT SEP.
CW
3
7
E
VAPORIZER
B
FEED TANK
D
2
PREHEATER
31
33
30
C